# EUROPEAN PATENT APPLICATION

(11) **EP 2 113 267 A1**
(43) Date of publication of application: **04.11.2009**
(21) Application number: 09010279.9
(22) Date of filing: 07.01.2008
(51) Int. Cl.: A61M 5/00, A61M 5/172, A61M 5/142

(54) **Automatic heart rate triggering of injection of radiopharmaceuticals for nuclear stress test**

(30) Priority: 10.01.2007 US 884295 P
(62) Divisional of application: 08713044.9
(71) Applicant: Mallinckrodt Inc., Hazelwood, MO 63042 (US)
(72) Inventor: Wilson, David, W., Loveland Ohio 45140 (US); Borgemenke, Elaine, Morrow Ohio 45152 (US)
(74) Representative: Chettle, Adrian John

(57) **Abstract**

A radiopharmaceutical injection system having a powered injector that includes radiation shielding, a biometric sensor, and a controller communicatively interconnected with both the powered injector and the biometric sensor. The controller may be configured to control the powered injector in response to a signal from the biometric sensor that indicates a biometric parameter is within a target range.

## Description

### FIELD OF THE INVENTION

The invention relates generally to medical fluid injectors and, more specifically, to medical fluid injectors responsive to biometric signal and/or remote control.

### BACKGROUND

This section is intended to introduce the reader to various aspects of art that may be related to various aspects of the present invention, which are described and/or claimed below. This discussion is believed to be helpful in providing the reader with background information to facilitate a better understanding of the various aspects of the present invention. Accordingly, it should be understood that these statements are to be read in this light, and not as admissions of prior art.

Cardiac stress testing is often used to assess cardiac function. In one form of cardiac stress testing, referred to as a nuclear stress test, a radiopharmaceutical is injected into a patient exhibiting an elevated heart rate. The patient's circulatory system carries the radiopharmaceutical to the patient's heart, and the resulting distribution of the radiopharmaceutical within different regions of the heart muscle is imaged. The images may be used to detect anomalies in blood flow to cardiac cells.

Conventionally, injections of radiopharmaceuticals during cardiac procedures are manually coordinated.
Generally, the patient Is exercising on a treadmill to reach peak heart rate when the healthcare provider injects the radiopharmaceutical. The procedure typically involves two individuals: one person monitoring a device to measure heart rate and another person performing the injection. Once the first person indicates that the patient has reached a target heart rate, the second person injects the radiopharmaceutical. Unfortunately, manually coordinated injections are expensive. Indeed, the labor costs associated with having two people perform the procedure can significantly impact the overall cost of the procedure.

### SUMMARY

Certain exemplary aspects of the invention are set forth below. It should be understood that these aspects are presented merely to provide the reader with a brief summary of certain forms the invention might take and that these aspects are not intended to limit the scope of the invention. Indeed, the invention may encompass a variety of aspects that may not be set forth below.

In certain aspects, the present invention generally relates to an injecting system that may be operated by a single person. In some embodiments, the injecting system includes a controller for remotely controlling a powered injector and, thereby, potentially enabling one person to both monitor an electrocardiograph (or other sensor(s)) and control an injection. Further, some embodiments may include a controller configured to automatically trigger an injection based on a signal from an electrocardiograph (or other sensor(s)), thereby potentially enabling a single technician to perform the test.

A first aspect of the present invention is directed to a radiopharmaceutical injection system having a biometric sensor, a controller, and a powered Injector that includes radiation shielding. The controller is communicatively interconnected (e.g., directly, indirectly, or wirelessly connected in a manner that enables information to be transmitted thereto and/or therefrom) with both the powered injector and the biometric sensor. Further, the controller is configured to control the powered injector in response to a signal from the biometric sensor that indicates a biometric parameter is within a target range.

A second aspect of the invention is directed to a radiopharmaceutical injection system having a powered injector and a remote controller communicatively interconnected to the powered injector. The powered injector includes a syringe receptacle and radiation shielding disposed about the syringe receptacle. In addition, the remote controller is configured to control the powered injector from a distance.

A third aspect of the invention is directed to a method of using a medical fluid injection system. In this method, a biometric parameter of a patient is measured and compared with a target. A powered injector of the system is remotely and/or automatically signaled to inject a medical fluid based on an outcome of the comparison.

Various refinements exist of the features noted above in relation to the various aspects of the present invention. Further features may also be incorporated in these various aspects as well. These refinements and additional features may exist individually or in any combination. For instance, various features discussed below in relation to one or more of the illustrated embodiments may be incorporated into any of the above-described aspects of the present invention alone or in any combination. Again, the brief summary presented above is intended only to familiarize the reader with certain aspects and contexts of the present invention without limitation to the claimed subject matter.

### BRIEF DESCRIPTION OF THE FIGURES

Various features, aspects, and advantages of the present invention will become better understood when the following detailed description is read with reference to the accompanying figures in which like characters represent like parts throughout the figures, wherein:

FIG.1 is a diagram of an imaging system;

FIG. 2 is a perspective view of a medical fluid injection system;

FIG. 2A is a perspective view of a controller of the medical fluid injection system of FIG.2;

FIG. 3 is a plan view of a powered injector;

FIG. 4 is a flow chart of a nuclear stress test; and

FIG. 5 is a flow chart of a cardiac stress test.

### DETAILED DESCRIPTION OF SPECIFIC EMBODIMENTS

One or more specific embodiments of the present invention will be described below. In an effort to provide a concise description of these embodiments, all features of an actual implementation may not be described in the specification. It should be appreciated that in the development of any such actual implementation, as in any engineering or design project, numerous implementation-specific decisions must be made to achieve the developers' specific goals, such as compliance with system-related and business-related constraints, which may vary from one implementation to another. Moreover, it should be appreciated that such a development effort might be complex and time consuming, but would nevertheless be a routine undertaking of design, fabrication, and manufacture for those of ordinary skill having the benefit of this disclosure.

When introducing elements of various embodiments of the present invention, the articles "a", "an", "the", and "said" are intended to mean that there are one or more of the elements. The terms "comprising", "including", and "having" are intended to be inclusive and mean that there may be additional elements other than the listed elements. Moreover, the use of "top", "bottom", "above", "below" and variations of these terms is made for convenience, but does not require any particular orientation of the components. As used herein, the term "coupled" refers to the condition of being directly or indirectly connected or in contact. Additionally, the phrase "in fluid communication" or "fluidly coupled" indicates that fluid and/or fluid pressure may be transmitted from one object to another.

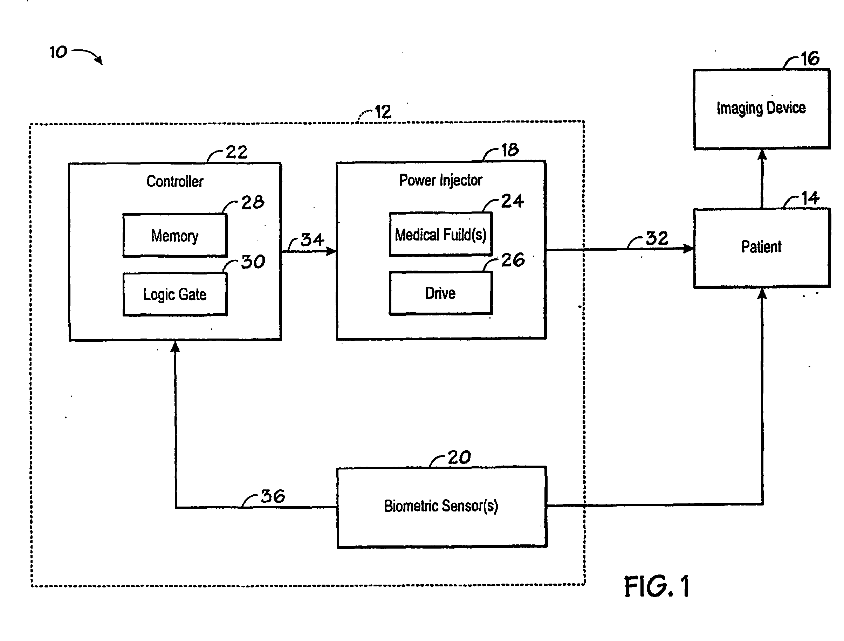

FIG. 1 depicts an exemplary diagnostic system 10 that may address one or more of the above-mentioned problems with conventional cardiac stress tests. The illustrated diagnostic system 10 includes an injection system 12, a patient 14, and an imaging device 16. As explained below, the injection system 12 may include components configured to automate the initiation of an injection in response to a biometric signal (e.g., a signal indicative of some aspect of a patient's physiological condition). Additionally, some embodiments may facilitate remote control of portions of the injection system 12, thereby potentially enabling a single technician to both monitor heart rate (and/or another biometric parameter) and initiate an injection. As explained below, various embodiments of the diagnostic system 10 may tend to reduce the cost of a cardiac stress test by reducing the number of participating technicians relative to conventional procedures.

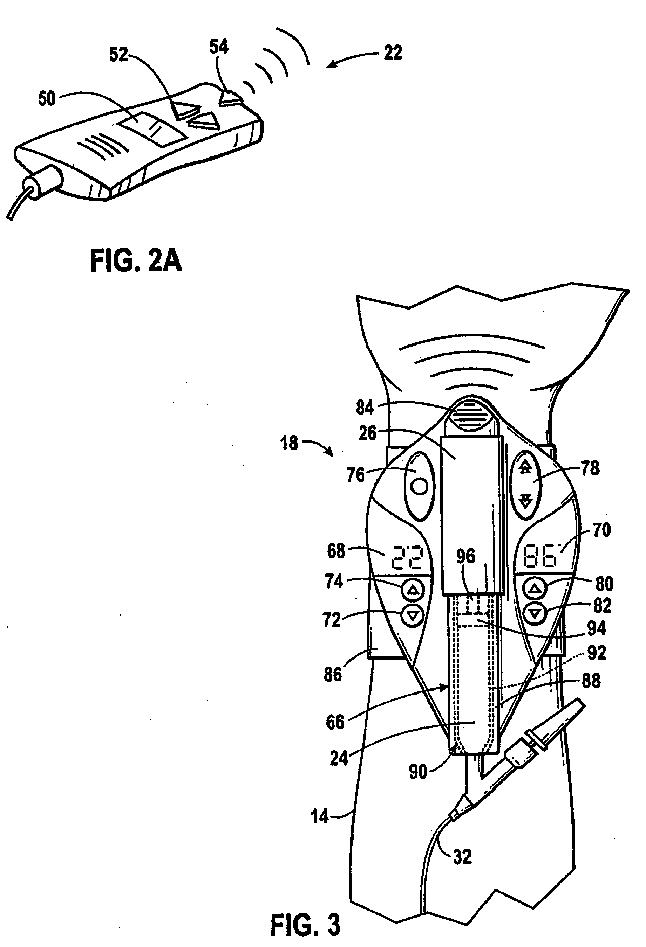

The exemplary injection system 12 includes a powered injector 18, a biometric sensor 20, and a controller 22. The powered injector 18 may include one or more medical fluids 24 (e.g., a gas, a liquid, a gel, a suspension, a Bingham plastic, etc.) and a drive 26. The medical fluids 24 may include a radiopharmaceutical, a contrast agent, a tagging agent, and/or saline. For example, the medical fluids 24 may include a container of technetium-99 solution and a container of saline. To attenuate radiation from radiopharmaceuticals, the powered injector 18 may include radiation shielding, as described below. The drive 26 may include an electric motor, pneumatic device, hydraulic device, thermo-mechanical device, piezoelectric device, or other device configured to convey the medical fluids 24. Features of an exemplary powered injector 18 are described below in reference to FIG. 3.

The biometric sensor 20 may include one or more of a variety of types of biometric sensors. For example, the biometric sensor 20 may Include a pulse oximeter (blood-oxygen monitor), a thermometer, a heart rate sensor, an electrocardiogram device, and/or a sphygmomanometer (blood pressure reading device).

The illustrated controller 22 includes a memory 28 and a logic gate 30. The memory 28 may include volatile and/or nonvolatile memory, and the logic gate 30 may include any of a variety of devices configured to perform a logical operation on one or more logic inputs and produce a logic output. For instance, the logic gate 30 may include, or be included in, a microprocessor, a digital signal processor, and/or an application-specific integrated circuit. In some embodiments, the logic gate may be constructed using electromagnetic relays, fluidics, optical, or mechanical devices configured to perform logical operations.

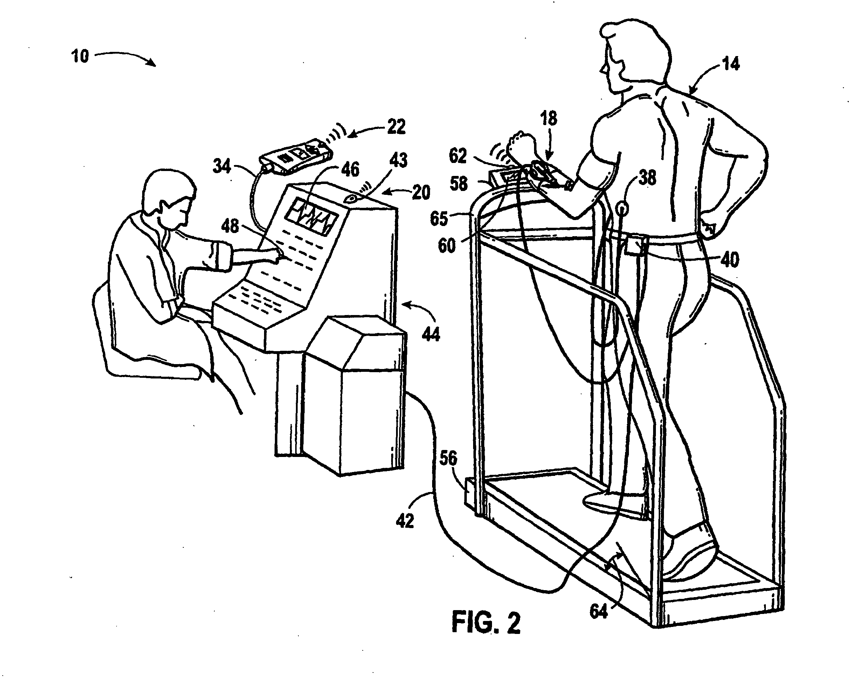

With reference to the other features of the diagnostic system 10, the patient 14 may be positioned on a piece of exercise equipment and have an elevated heart rate. For example, a patient 14 may be exercising on a track, a treadmill (such as the one described below in reference to FIG. 2), an exercise bike, an elliptical machine, a stair-stepper machine, or other device configured to elevate the patient's heart rate. In some embodiments, one or more chemicals may be administered to the patient to alter a given biometric parameter (e.g., increase the patient's heart rate). The imaging device 16 may include a projection radiography system, a fluoroscopy system, a tomography system, a magnetic resonance imaging system, and/or an ultrasound system.

As assembled, the powered injector 18 may be in fluid communication with the patient 14 via a fluid conduit 32. The fluid conduit 32 may include tubing and/or a hypodermic needle positioned in the patient 14. The powered injector 18 may be communicatively interconnected to the controller 22 by a control signal path 34, and the controller 22 may be communicatively interconnected with the biometric sensor 20 by a biometric signal path 36. The control signal path 34 and/or the biometric signal path 36 may include a variety of components configured to convey information, such as wires, conductive traces, and/or wireless interfaces configured to wirelessly interconnect devices.

Various components of the injection system 12 may be integrated with one another (e.g., share a common housing or have housings that are configured to connect to one another). For instance, the controller 22 may be integrated with the powered injector 18 and/or the biometric sensor 20.

In operation, the controller 22 may signal the powered injector 18 to start and/or stop an injection based on feedback from the biometric sensor 20. For example, the biometric sensor 20 may include an electrocardiogram device that measures heart rate, and the controller 22 may be configured to initiate an injection when the biometric sensor 20 indicates that the patient's heart rate is within a target range stored in memory 28. The range stored in memory 28 may be heart rates at or above a peak heart rate calculated for the patient 14. In some embodiments, the controller 22 may be configured to calculate the target range based on patient parameters, such as age, weight, height, and/or gender. The range may generally correspond to values greater than a target, greater than or equal to a target, values within a minimum and maximum value (inclusive or exclusive of the minimum and/or maximum value), values less than a target, values less than or equal to a target, or values approximately equal to a target.

The powered injector 18 may be controlled by the controller 22 from a remote location (e.g., from a distance greater than 2 or 3 feet). To Indicate when to exercise remote control, the controller 22 and/or biometric sensor 20 may include an audio and/or visual indicator to signal when a patient's heart rate is within the target range. In response to such an indication, a technician may remotely signal the powered injector 18 to start injecting the medical fluids. To this end, the technician may press a button or flip a switch on the controller 22 indicating a desire to start or stop an injection, and the controller 22 may transmit a corresponding signal on the control signal path 34, which, as mentioned above, may be wired or wireless.

In some embodiments, the powered injector 18 may be automatically controlled by the controller 22 based on signals from the biometric sensor 20. For instance, the logic gate 30 may compare the signal from the biometric sensor 22 to a target value or target range stored in memory 28, such as a target heart rate range. The logic gate 30 may determine whether the patient's heart rate or other biometric parameter is within the target range or generally equal to the target value. In the event that the target condition is met, the logic gate 30 may output a signal to initiate an injection, e.g., by transmitting a signal to start an injection via the control signal path 34. Similarly, the controller 22 may signal the powered injector 18 to stop an injection based on a signal from the biometric sensor 20 or some other parameter, such as a duration of time. In some embodiments, a technician may override the automatic aspects of the present embodiment and manually adjust (e.g., start, stop, pause, or modify) an injection by, for example, pressing a button or using some other user interface on the controller 22 and/or the powered injector 18.

In response to a signal from the controller 22 to start an injection, the drive 26 on the powered injector 18 may begin pumping or conveying the medical fluids 24 into the patient 14. For instance, the drive 26 may elevate the pressure of a container holding the medical fluids 24 (e.g., the drive 26 may drive a plunger through a barrel of a syringe), and, as a result, the medical fluids 24 may flow through the fluid conduit 32 into the patient 14. In some embodiments, the drive 26 may sequentially convey medical fluids 24, e.g., a radiopharmaceutical followed by saline. In response to a signal from the controller 22 to stop an injection, user input to this effect, or a determination that the injection is complete, the drive 26 may cease pumping or conveying the medical fluids 24 into the patient 14.

During the injection, an audible tone, visual indication, or both may alert a technician that an injection has begun or is in process. Once the injection has been completed, a different audible tone, visual indication or combination thereof may alert the technician that the Injection is complete.

Advantageously, in some embodiments, a single technician may both monitor the biometric sensor 20 and initiate an injection, e.g., remotely and/or automatically. Further, the technician may be positioned further from the radiopharmaceutical than in conventional imaging systems, and radiation from the radiopharmaceutical may be attenuated over the distance.

FIG. 2 is a perspective view illustrating additional details of the injection system 10. The biometric sensor 20 includes a leads 38 (e.g., 12 leads), an intermediate signal processor 40, an intermediate signal path 42, a wireless interface 43 and a sensor signal processor 44. The leads 38 are placed at various points on the body of the patient 14 to measure electric potentials therebetween. The leads 38 may couple to the intermediate signal processor 40, which may be configured to amplify and/or transmit signals detected via the leads 38. The intermediate signal path 42 may connect the intermediate signal processor 40 to the sensor signal processor 44, which may be configured to calculate various biometric parameters based on signals received via the intermediate signal path 42. For instance, the sensor signal processor 44 may be configured to calculate a patient heart rate. The sensor signal processor 44 may include a display 46 and user input 48 for interacting with a technician. In some embodiments, the display 46 may display a trace representative of a patient's heart beat.

Referring to FIG. 2A, the illustrated controller 22 includes a display 50, a user input 52, and a wireless interface 54. The controller 22 may be characterized as a handheld device (e.g., a device sized to fit in an average person's hand). The display 50 may be configured to display patient data, such as biometric data based on signals from the biometric sensor 20, the patient's age, the patient's weight, the patient's height, the patient's medical history, and/or instructions (e.g., instructions stored on a network). The display 50 may also be configured to display data relevant to an injection, such as data relevant to the potency of a radiopharmaceutical in the medical fluid 24, estimated time until an injection based on a current heart rate, duration of an injection, time until an injection is complete, a rate of injection, a volume of injection, a target parameter to trigger an injection, a target range or value for that parameter, and/or a sequence of injection. The user input 52 may be configured to enable a technician to manually initiate an injection, stop an injection, adjust an injection, and/or enter a target parameter, range, or value.

The controller 22 may communicate with the sensor signal processor 44 via a biometric signal path 34 that is embodied by a cable or a wireless link (e.g., between wireless interfaces 43, 54). In some embodiments, the controller 22 may not communicate with the biometric sensor 20, and a technician may manually operate the controller 22 in response to signals from the biometric sensor 20, e.g., in response to information displayed on the display 46.

The wireless interfaces 43, 54 may be configured to communicate with other wireless interfaces in accordance with a variety of protocols, such as WiFi, WAP, Bluetooth, and/or Wibree, among others. The wireless interfaces 43, 54 may also be configured to transmit and/or receive data via an Infrared or optical link. In some embodiments, the controller 22 may be connected to the powered injector 18 by a cable. In such an embodiment, the cable may be longer than 3 feet, 4 feet, 5 feet, 6 feet, 7 feet, 8 feet, 9 feet, or 10 feet to facilitate remote control of the powered injector 18.

The illustrated diagnostic system 10 includes a treadmill 56 having a treadmill controller 58, a treadmill display 60, and a wireless interface 62. The speed, resistance, and/or angle of inclined 64 of the treadmill 56 may be modulated to appropriately physically stress the patient 14. In some embodiments, the treadmill controller 58 may control one or more of these parameters based on patient data, such as age, weight, height, and/or gender. Alternatively, or additionally, the treadmill controller 58 may be configured to exercise control over one or more of these parameters based on a signal from the controller 22, the biometric sensor 20, and/or input from the patient 14. The treadmill display 60 may be configured to display a biometric parameter of the patient 14 (such as a heart rate), a time remaining in the test, a speed, an angle of inclined 64, and/or a resistance level to the patient 14. To this end, the treadmill controller 58 may communicate with the controller 22, the biometric sensor 20, and/or the powered injector 18 via the wireless interface 62 using one or more of the above-mentioned protocols. In some embodiments, the treadmill controller 58 may be linked to the controller 22, the biometric sensor 20, and/or the powered injector 18 via a cable or conductive trace.

Various components of the injection system 12 may be integrated with the treadmill 56 (or some other type of exercise equipment, as mentioned above). For instance, all or part of the biometric sensor 20 may be integrated with the treadmill 56, e.g., the treadmill 56 may include a galvanometer with leads disposed on a handle 65. In another example, the powered injector 18 may be removeably coupled to the treadmill 56. For instance, the powered injector 18 may be in fluid communication with the patient 14 via a fluid conduit that is long enough to enable the patient 14 to exercise while the powered Injector 18 remains coupled to the treadmill 56. Further, the controller 22 may be integrated with the treadmill controller 58 on the treadmill 56.

FIG. 3 is a plan view of the powered injector 18. In addition to the medical fluids 24 and drive 26, the powered injector 18 may include a syringe receptacle 66, displays 68, 70, buttons 72, 74, 76, 78, 80, 82, a wireless interface 84, and a strap 86. The illustrated powered injector 18 may be characterized as a patient-borne powered injector, a wearable powered injector, and/or a hands-free powered injector.

The syringe receptacle 66 includes shielding 88 and a syringe 90 having a barrel 92, a plunger 94, and a push rod 96. The shielding 88 is configured to attenuate radiation from a radiopharmaceutical in the syringe 90 and may include radiation shielding materials, such as tungsten impregnated plastic, tungsten, lead, plastic-coated lead, leaded glass, or a combination of these materials. The shielding 88 may substantially envelope the syringe 90 to attenuate radiation from the medical fluids 24. The illustrated syringe 90 is disposed largely within the shlelding 88 and is loaded with the medical fluid 24. Some embodiments may include a syringe receptacle 66 configured to hold multiple syringes 90, such as a syringe holding a radiopharmaceutical and a syringe holding saline. Alternatively, some embodiments may include a two-stage syringe configured to sequentially deliver two different medical fluids 24. The drive 26 is coupled to the syringe 90 via the push rod 96. During an injection, the drive 26 pushes the push rod 96 and plunger 94 through the barrel 92 of the syringe 90. As a result, the medical fluid 24 may be pumped through the fluid conduit 32 and Into the patient 14. In some embodiments, the drive 26 may interface directly with the plunger 94.

The displays 68, 70 and buttons 72, 74, 76, 78, 80, 82 may Interface with users of the powered injector 18. For instance, the display 70 may display a target heart rate, and the display 68 may display a volume or dosage to be injected. The buttons 72, 74, 76, 78, 80, 82 may be used to manipulate which values the displays 68, 70 display and various injection parameters, such as a rate of injection, a volume of injection, a duration of injection, an injection flow rate profile, and/or a sequence of injection. Additionally, the displays 68, 70 and buttons 72, 74, 76, 78, 80, 82 may be used to view and enter data relevant to the potency of a radiopharmaceutical, such as the volume of the medical fluid 24, the time at which the medical fluid 24 expires, the time at which the medical fluid 24 was loaded into the powered injector 18, and/or a half-life of the medical fluid 24.

The strap 86 may be configured to attach the powered injector 18 to the wrist of the patient 14. To this end, the strap 86 may include Velcro, a buckle, buttons, elastic materials, and/or adhesives to secure the strap 86 to the patient 14. In some embodiments, the powered injector 18 may be coupled to other parts of the patient 14, such as the upper arm, the chest, the thigh, the calf, the head, the neck, or other portion of the patient 14. Alternatively, the powered injector 18 may be removeably or permanently attached to the treadmill 56, the biometric sensor 20, or a stand placed near the treadmill 56. In another embodiment, the powered injector 18 may include a clothing mount, such as a clip, configured to mount on the patient's clothing (e.g., belt, shirt, pants, gown, etc.).

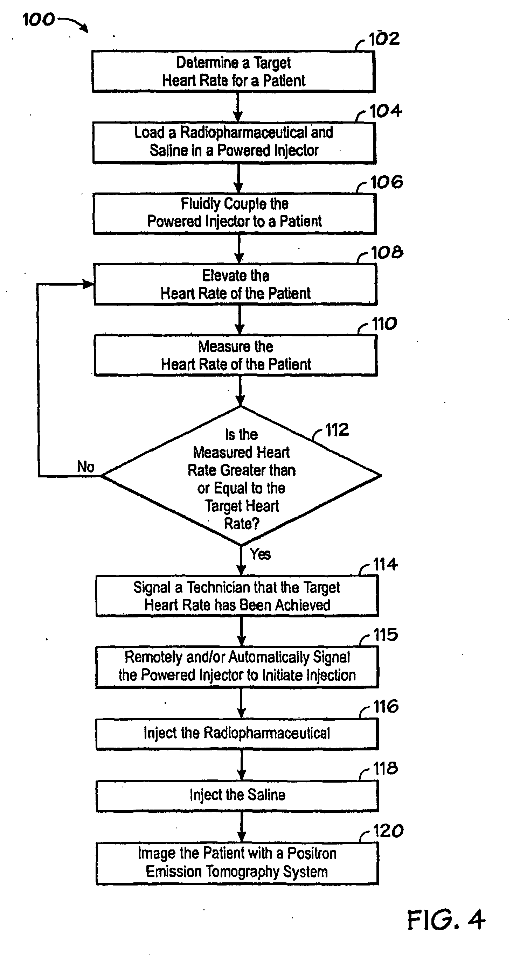

FIG. 4 is a flowchart of an exemplary nuclear stress test 100. The illustrated nuclear stress test 100 begins with determining a target heart rate for a patient, as depicted by block 102, and loading a radiophamaceutical and saline into a powered injector, as depicted by block 104. Loading the radiopharmaceutical may include extracting a desired isotope from a radiopharmaceutical generator. In some embodiments, the radiopharmaceutical generator may be in a separate room from other components of the imaging system 10 to reduce exposure to radiation. Alternatively, the radioisotopes may be produced in a cyclotron. Next, the powered injector maybe fluidly coupled to the patient, as depicted by block 106, and the heart rate of the patient may be elevated, as depicted by block 108. While elevating the patient's heart rate, the patient's heart rate may be measured, as depicted by block 110, and compared to a target heart rate, as depicted by block 112. If the patient's heart rate is not greater than or equal to the target heart rate, the acts depicted by blocks 108, 110, 112 may be repeated. That is, the patient may continue to exercise until the target heart rate is reached. On the other hand, if the patient's heart rate is greater than or equal to the target heart rate, an audible or visual signal may indicate to a technician that the target heart rate has been achieved, as depicted by block 114. Next, the technician may remotely control the powered injector to initiate an injection and/or a signal may be automatically transmitted to the powered Injector to initiate the injection, as depicted by block 115. In response to the signal to initiate injecting, the powered injector may inject the radiopharmaceutical, as depicted by block 116, and, then, inject the saline, as depicted by block 118. To diagnose the patient, the patient may be imaged with a positron emission tomography system, as depicted by block 120.

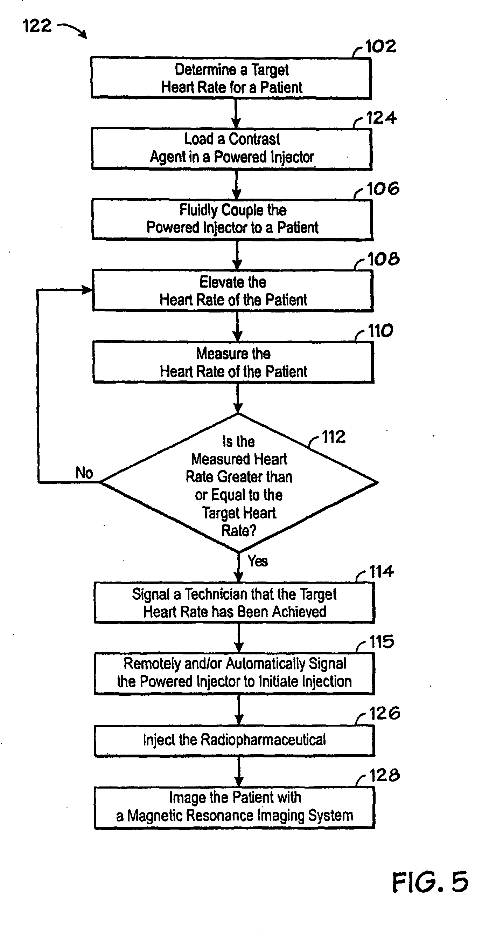

FIG. 5 is a flowchart of an exemplary cardiac stress test 122. The cardiac stress test 122 includes many of the same steps as the nuclear stress test 100, e.g., the steps depicted by blocks 102, 104, 106, 108, 110, 112, 114, and 115. Additionally, cardiac stress test 122 includes a number of steps that are different from corresponding steps in the nuclear stress test 100. For instance, a contrast agent is loaded into the powered injector, as depicted by block 124, and injected in the patient, as depicted by block 126. Additionally, the patient is imaged with a magnetic resonance imaging system, as depicted by block 128. In some embodiments, the cardiac stress test 122 may be performed in conjunction with the nuclear stress test 100.

## Claims

1. A radiopharmaceutical injection system comprising:
a powered injector comprising a syringe receptacle and radiation shielding disposed at least partially about the syringe receptacle; and
a remote controller communicatively interconnected to the powered injector, wherein the remote controller is configured to control the powered injector from a distance.

2. The system of claim 1, wherein both the remote controller and the powered injector each comprise a wireless interface configured to wirelessly interconnect the powered injector and the remote controller.

3. The system of claim 2, wherein each wireless interface comprises a Bluetooth wireless interface.

4. The system of claim 1, wherein the remote controller is communicatively interconnected to the powered injector by a cable that is longer than 3 feet.

5. The system of any of claims 1-4, wherein the remote controller comprises a user input device and is configured to signal the powered injector to initiate an injection in response to a signal from the user input device.

6. The system of any of claims 1-5, comprising a biometric sensor.

7. The system of claim 6, wherein the biometric sensor is communicatively interconnected to the remote controller, and wherein the remote controller is configured to signal the powered injector to start an injection in response to a signal from the biometric sensor.

8. The system of claim 7, wherein the biometric sensor comprises a heart rate sensor and the target range is heart rates greater than or equal to a predetermined heart rate.

9. The system of claim 8, wherein the controller is configured to signal the powered injector to initiate an injection in response to a signal from the biometric sensor that indicates a patient's heart rate is greater than or equal to a target heart rate.

10. The system of any of claims 1-9, wherein the biometric sensor comprises an electrocardiograph.

11. The system of any preceding claim, wherein the powered injector comprises a wearable powered injector.

12. The system of any preceding claim, wherein the powered injector comprises a strap configured to secure the powered injector to a patient.
